# EUROPÄISCHE PATENTSCHRIFT

(11) **EP 2 719 465 B1**
(45) Veröffentlichungstag und Bekanntmachung des Hinweises auf die Patenterteilung: **28.03.2018**
(21) Anmeldenummer: 13184893.9
(22) Anmeldetag: 18.09.2013
(51) Int. Cl.: B05B 11/00, A61M 15/08

(54) **Spender mit Kindersicherung**
Dispenser with child-proof protection system
Distributeur avec sécurité enfants

(30) Priorität: 10.10.2012 DE 102012218434
(43) Veröffentlichungstag der Anmeldung: 16.04.2014
(73) Patentinhaber: Aptar Radolfzell GmbH, 78315 Radolfzell (DE)
(72) Erfinder: Szymiczek, Christoph, 78224 Singen (DE); Greiner-Perth, Jürgen, 78244 Gottmadingen (DE)
(74) Vertreter: Patentanwaltskanzlei Cartagena

(56) Entgegenhaltungen:
- WO-A1-2005/107955
- WO-A1-2010/089562
- WO-A1-2012/103740
- DE-A1- 3 342 883
- DE-A1-102010 011 761
- FR-A1- 2 876 676
- US-A- 3 770 168
- US-A- 4 368 830
- US-A1- 2003 106 901
- US-A1- 2007 080 174
- US-A1- 2009 242 502
- US-A1- 2009 255 958

## Beschreibung

### Anwendungsgebiet und Stand der Technik

Die Erfindung betrifft einen Spender zum Austrag eines flüssigen oder pulverförmigen Mediums mit einem Mediumspeicher, mit einer Austragöffnung und mit einer Fördereinrichtung zur Förderung von Medium vom Mediumspeicher zur Austragöffnung. Dabei weist ein gattungsgemäßer Spender eine Betätigungshandhabe der Fördereinrichtung auf, die gegenüber dem Mediumspeicher in einer Betätigungsrichtung verlagerbar ist, um hierdurch einen Austrag des Mediums zu bewirken.

Gattungsgemäße Spender sind aus dem Stand der Technik allgemein bekannt. Rein beispielhaft wird auf die DE 10 2004 050 679 A1 verwiesen, aus der ein Nasalspender mit den gattungsgemäßen Merkmalen bekannt ist.

Gattungsgemäße Spender dienen dem Austrag pharmazeutischer Medien. Es ist daher häufig gewünscht, die Zugänglichkeit des Mediums für Kinder zu erschweren, so dass diese nicht versehentlich beim Spielen mit möglicherweise schädlichen Medien in Kontakt kommen.

Aus dem Stand der Technik sind verschiedene Möglichkeiten für kindergesicherte Spender bekannt. So schlägt die DE 10 2009 049 903 A1 beispielsweise zum Einen Spender vor, die bei aufgesetzter Kappe nicht betätigt werden können, da ihre Betätigungshandhabe durch die Kappe blockiert wird. Zum Anderen nennt die genannte Schrift Kappengestaltungen, die nur durch ungewöhnliche Bewegungsabläufe vom jeweiligen Spender zu trennen sind.

Viele der bekannten Gestaltungen von kindergesicherten Spendern betreffen Spender, die bereits originär als kindergesicherte Spender konzipiert worden sind. Derartige kindergesicherte Spender sind jedoch in der Herstellung vergleichsweise teuer, da ihre Teilkomponenten meist nicht für andere möglicherweise nicht kindergesicherte Spender verwendbar sind, so dass eigene Werkzeuge für mit meisten Teilkomponenten solcher kindergesicherter Spender erforderlich sind. Einen Spender mit einer Sicherungsvorrichtung gemäß dem Oberbegriff von Anspruch 1 ist in WO2010/089562 offenbart.

### Aufgabe und Lösung

Aufgabe der Erfindung ist es, hierzu eine Alternative zur Verfügung zu stellen, nämlich Gestaltungen kindergesicherter Spender, die in nur geringem Maße den Grundaufbau des Spenders betreffen, so dass der Spender bei nur geringfügigen Anpassungen sowohl als kindergesicherter als auch als nicht kindergesicherter Spender hergestellt werden kann.

Gemäß einer ersten Variante der Erfindung ist bei einem gattungsgemäßen Spender eine Sicherungsvorrichtung vorgesehen, die aus mindestens zwei und vorzugsweise genau zwei Teilschalen besteht, die in einem Sicherungszustand durch mindestens eine zum wiederholten Entkoppeln und Koppeln ausgebildete Kopplungsvorrichtung derart miteinander verbunden sind, dass sie gegeneinander unbeweglich sind. Dabei ist die Sicherungsvorrichtung an der Fördereinrichtung des Spenders in einer Sicherungsposition derart anbringbar, dass im Sicherungszustand der Sicherungsvorrichtung mit fest verbundenen Teilschalen die Zugänglichkeit und/oder die Beweglichkeit der Betätigungshandhabe verhindert wird. Erst durch Lösen der Kopplungsvorrichtung ist ein Freigabezustand der Sicherungsvorrichtung erzielbar, in dem die Teilschalen gegeneinander beweglich sind, so dass die zuvor die Fördereinrichtung umgebendende Sicherungsvorrichtung von der Fördereinrichtung trennbar ist und so die Zugänglichkeit und/oder Beweglichkeit der Betätigungshandhabe dadurch erreichbar ist.

Ebenso wie bei gattungsgemäßen Spendern ist auch bei einem erfindungsgemäßen Spender vorgesehen, dass dieser den genannten Mediumspeicher aufweist, in welchem das vorzugsweise flüssige, möglicherweise jedoch auch pulverförmige Medium vor dem Austrag vorliegt. Die Fördereinrichtung eines solchen Spenders verbindet den Mediumspeicher mit einer Austragöffnung, durch die hindurch das Medium in eine umgebende Atmosphäre und somit insbesondere Körperteile eines Patienten abgegeben werden kann. Diese Fördereinrichtung ist mittels einer Betätigungshandhabe betätigbar, wobei die Betätigungshandhabe vorzugsweise entlang eines definierten und insbesondere linearen Pfades gegenüber dem Mediumspeicher verlagerbar ist, um den Austragvorgang zu bewirken. Eine Möglichkeit zur Ausgestaltung einer solchen Fördereinrichtung ist die Ausgestaltung als Kolbenpumpe, die durch Druckbeaufschlagung einer Teilcharge des Mediums dieses durch die Austragöffnung hindurch nach außen treibt.

Die Betätigungshandhabe ist vorzugsweise in Art einer zur Auflage eines oder zweier Finger vorgesehenen Fingerauflage ausgebildet.

Die gemäß dieser ersten Variante der Erfindung vorgesehene Sicherungsvorrichtung weist genannte zwei Teilschalen auf, die in ihrem gekoppelten Sicherungszustand die Unbeweglichkeit oder Unzugänglichkeit der Betätigungshandhabe bewirken. Sie können hierzu die Fördereinrichtung und die Betätigungshandhabe als Ganzes derart umgeben, dass vor dem Trennen der Sicherungsvorrichtung von der Fördereinrichtung von außen nicht einmal das Vorhandensein der Betätigungshandhabe erkennbar ist. Bei einer vereinfachten Gestaltung ist die Betätigungshandhabe zwar bei angebrachter Sicherungsvorrichtung zu erkennen, jedoch entweder nicht zugänglich oder aber durch die Sicherungsvorrichtung vorzugsweise formschlüssig derart blockiert, dass sie nicht in ausreichendem Maße gegenüber dem Mediumspeicher verlagerbar ist, um dadurch eine signifikante Förderung von Medium zu bewirken. Die zwei Teilschalen können wiederholt aneinandergekoppelt und voneinander getrennt werden, so dass jeweils vor der Verwendung des Spenders die Entkopplung der Kopplungsvorrichtung und die Abnahme der Sicherungsvorrichtung stattfindet, während nach erfolgter Verwendung des Spenders die beiden Teilschalen in der Sicherungsposition wieder aneinandergekoppelt werden und somit die Handhabung durch Kinder verhindern.

Die Sicherungsvorrichtung ist derart an die Formgebung des Spenders angepasst, dass sie nicht ohne vorheriges Entkoppeln abgezogen werden kann.

Im Sicherungszustand der Sicherungsvorrichtung, in dem die Teilschalen unbeweglich gegeneinander sind, bilden diese eine zumindest einseitig offene Hülse, die den Spender nur partiell im Bereich der Fördereinrichtung und gegebenenfalls bis hin zur Austragöffnung umgibt. Bei einer Gestaltung, bei der die Austragöffnung nicht mit umgeben sein soll, kann diese durch die beiden Teilschalen gebildete Hülse auch beidseitig offen sein.

Die beiden Teilschalen der Sicherungsvorrichtung weisen vorzugsweise etwa die gleiche Größe auf, wobei hierunter zu verstehen ist, dass die Teilschalen die vorzugsweise etwa rotationssymmetrische Fördervorrichtung jeweils über einen Winkel von etwa 180° (+/- 10°) umgeben. Hiervon abweichend sind allerdings auch Gestaltungen denkbar, bei denen deutlich unterschiedliche Größen vorgesehen sind, bei denen die kleinere der Teilschalen nur einen Winkelbereich zwischen 90° und 170° umgibt.

Rein technisch funktioniert die Gestaltung mit nur einer Kopplungsvorrichtung, da die Teilschalen vorzugsweise auch im entkoppelten Zustand nicht vollständig voneinander getrennt sind, sondern lediglich in definierter Weise gegeneinander bewegt werden können. Hierzu kann insbesondere eine Scharnierverbindung, insbesondere eine Verbindung durch ein Filmscharnier, zwischen den Teilschalen vorgesehen sein. Grundsätzlich können jedoch auch zwei vollständig getrennte Teilschalen und vorzugsweise zwei einander gegenüberliegenden Kopplungsvorrichtungen vorgesehen sein, die nur im Sicherungszustand in Verbindung zueinander sind.

Für Kinder ist es insbesondere bei einer Gestaltung der Sicherungsvorrichtung, bei der diese auch die Austragöffnung verdeckt, schwer zu durchschauen, dass die Sicherungsvorrichtung abgenommen werden muss und dass hierfür zunächst eine Entkopplung der Teilschalen erforderlich ist. Insbesondere wird die Sicherheit noch dadurch erhöht, dass die Kopplungsvorrichtung vorzugsweise derart ausgebildet ist, dass sie durch Kinder schwer zu handhaben ist.

Vorzugsweise weist die mindestens eine Kopplungsvorrichtung zwei formschlüssig wirkende Kopplungsabschnitte an den beiden Teilschalen auf, die durch abschnittsweises Verformen einer oder beider Teilschalen aus dem gekoppelten in den entkoppelten Zustand überführt werden können. Insbesondere kann es hierfür zweckmäßigerweise erforderlich sein, zumindest einen Kopplungsabschnitt einer oder beider Teilschalen in einer Radialrichtung auszulenken.

Dennoch wird es als vorteilhaft angesehen, wenn mindestens zwei getrennt handhabbare Kopplungseinrichtungen auf der gleichen Seite der Sicherungsvorrichtung vorgesehen sind, die eine komplexere Relativbewegung von Teilabschnitten der Teilschalen zum Zwecke des Entkoppelns erforderlich machen. So kann einerseits vorgesehen sein, dass durch Verformung von Kopplungsabschnitte beider Teileinrichtungen in gleicher Richtung die Entkoppelbarkeit gegeben sein kann. Hierunter ist zu verstehen, dass im Bereich einer Kontaktlinie der Teilschalen zwei Kopplungseinrichtungen versetzt zueinander vorgesehen sind, die jeweils durch Verformen von an den beiden Teilschalen vorgesehenen Kopplungsabschnitte in identischer Richtung entkoppelt werden. Alternativ hierzu könnte auch vorgesehen sein, dass zwei Kopplungsabschnitte an der gleichen Teilschale in gegenläufige Richtungen verlagert werden müssen, um die Entkoppelbarkeit zu ermöglichen. So könnte einer dieser Teilabschnitte radial in Richtung der Fördereinrichtung und der andere Teilabschnitt radial von der Fördereinrichtung weg bewegbar sein.

Eine ebenfalls zweckmäßige Art zur Herstellung einer durch Kinder schwer handhabbaren Kopplungsvorrichtung ist dadurch erzielbar, dass die Kopplungsvorrichtung eine Kopplungsdurchbrechung an einer Teilschale und einen die Kopplungsdurchbrechung in gekoppeltem Zustand durchdringenden Kopplungsfortsatz an der anderen Teilschale aufweist, wobei der Kopplungsfortsatz derart geformt ist, dass er zum Hindurchführen durch die Kopplungsdurchbrechung zum Zwecke des Entkoppelns tordiert werden muss. Es wurde festgestellt, dass die Torsionsbewegung des Kopplungsfortsatzes eine für Kinder schwer zu durchschauende und motorische zu anspruchsvolle Handhabung darstellt.

Hinsichtlich aller Varianten ist vorzugsweise vorgesehen, dass alle beschriebenen Teil, insbesondere alle Teile der Sicherungsvorrichtungen, als Kunststoffteile ausgebildet sind.

Da ein erfindungsgemäßer Spender zum Austrag von pharmazeugischen Flüssigkeiten dient, ist er im Auslieferungszustand vorzugsweise mit einer solchen befüllt. Insbesondere ist die Verwendung eines erfindungsgemäßen Spender für pharmazeutische Flüssigkeiten vorgesehen, hinsichtlich derer die Gefahr für Kinder besonders groß ist. Es kann sich hier beispielsweise um Schmerzmittel handeln. Jedoch eignet sich ein erfindungsgemäßer Spender für alle anderen Arten flüssiger und pulverförmiger verschreibungspflichtiger Medikamente.

### Kurzbeschreibung der Zeichnungen

Weitere Aspekte und Vorteile der Erfindung ergeben sich außer aus den Ansprüchen auch aus der nachfolgenden Beschreibung bevorzugter Ausführungsbeispiele der Erfindung. Dabei zeigen:
- Fig. 1a und 1b: einen erfindungsgemäßen Spender gemäß der Erfindung in einer ungeschnittenen und einer teilgeschnittenen Darstellung,
- Fig. 2: die Sicherungsvorrichtung des Spenders der Fig. 1a und 1b im entkoppelten Zustand,
- Fig. 3a und 3b: Varianten zum Spender der Fig. 1a, 1b und 2,
- Fig. 4 und 5: einen Spender gemäß einer nicht erfindungsgemäßen Variante in einer teilgeschnittenen Ansicht eines gesicherten Zustandes des Spenders und einer ungeschnittenen Ansicht des entsicherten Zustandes,
- Fig. 6: eine Varianten zum Spender der Fig. 4 und 5,
- Fig. 7a und 7b: einen Spender gemäß einer nicht erfindungsgemäßen Variante in einer teilgeschnittenen und einer ungeschnittenen Darstellung und
- Fig. 8: eine Schnittdarstellung durch die Sperrvorrichtung des Spenders der Fig. 7a und 7b.

### Detaillierte Beschreibung der Ausführungsbeispiele

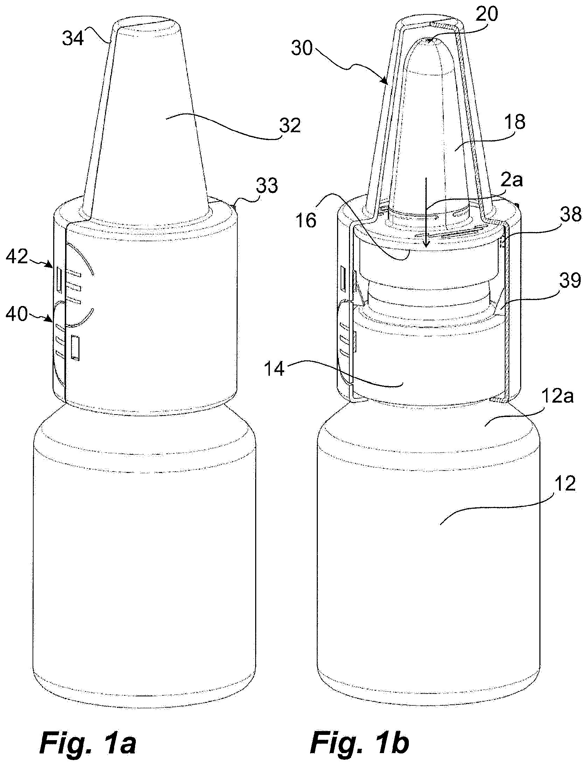

Die Fig. 1a und 1b zeigen eine Ausführungsform der Erfindung. Bezug nehmend auf die teilgeschnittene Darstellung der Fig. 1b findet hier ein Spender Verwendung, der in Hinblick auf seinen Grundaufbau mit bekannten Spendern vergleichbar ist. Dieser Grundaufbau umfasst einen Mediumspeicher 12, eine an einem Hals 12a des Mediumspeichers 12 angebrachte Fördereinrichtung 14 mit einer Betätigungshandhabe 16 zur Betätigung der Fördereinrichtung 14 und eine vorliegend am distalen Ende einer Nasenolive 18 vorgesehene Austragöffnung 20. Durch Verlagerung der Betätigungshandhabe 16 in Richtung des Pfeils 2a kann eine nicht näher dargestellte innerhalb der Fördereinrichtung 14 vorgesehene Kolbenpumpe betätigt werden, die flüssiges Medium aus dem Mediumspeicher 12 zur Austragöffnung 20 fördert.

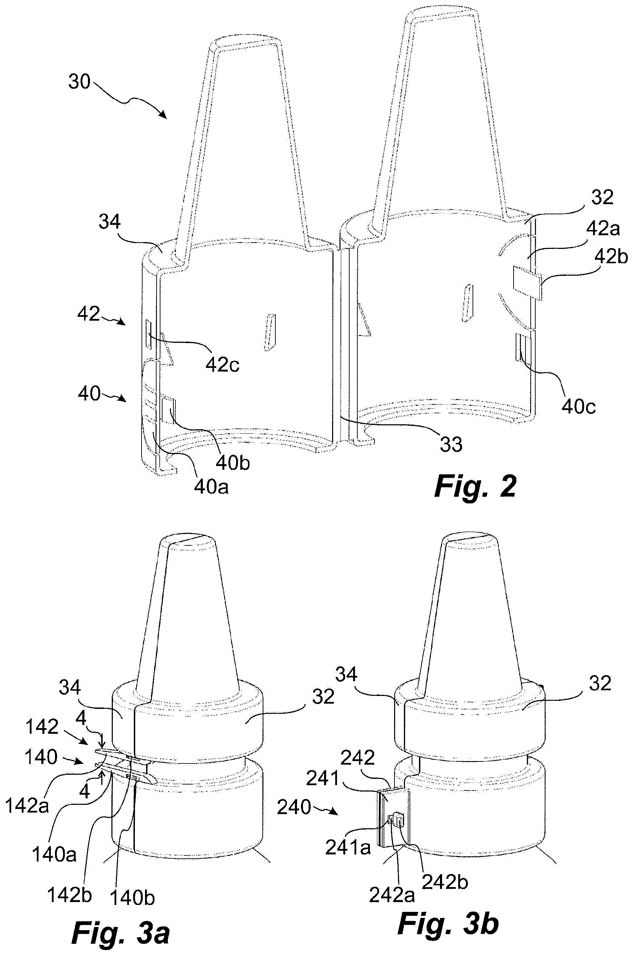

Um die missbräuchliche Verwendung des Spenders durch Kinder zu verhindern, ist eine Sicherungsvorrichtung 30 vorgesehen. Diese Sicherungsvorrichtung 30 verfügt über zwei Teilschalen 32, 34, die durch ein Filmscharnier 33 miteinander verbunden sind. In der durch Fig. 2 verdeutlichten Weise gestattet es dieses Filmscharnier 33, die Teilschalen 32, 34 gegeneinander zu verschwenken, um den geöffneten Zustand der Sicherungsvorrichtung 30 herzustellen. Im geschlossenen Zustand, der in den Fig. 1a und 1b dargestellt ist, sind die Teilschalen 32, 34 gegenüber dem Zustand der Fig. 2 um 180° verschwenkt und durch zwei Kopplungsvorrichtungen 40, 42 im geschlossenen Zustand gesichert.

Die Kopplungsvorrichtungen 40, 42 sind baugleich ausgebildet, unterscheiden sich jedoch hinsichtlich ihrer Verteilung auf die Teilschalen 32, 34. Im Falle der Kopplungsvorrichtung 40 ist ein gegenüber der Teilschale 34 elastisch auslenkbarer Kopplungsabschnitt 40a mit einem Klinkenabschnitt 40b an der Teilschale 34 vorgesehen, während an der anderen Teilschale 32 eine Ausnehmung 40c für den Klinkenabschnitt 40b vorgesehen ist. Im Falle der zweiten Kopplungsvorrichtung 42 ist dagegen der elastisch auslenkbare Abschnitt 42a mit dem Klinkenabschnitt 42b an der Teilschale 32 vorgesehen, während die korrespondierende Ausnehmung 42c an der zweiten Teilschale 34 vorgesehen ist.

Diese Anordnung führt dazu, dass es zum Trennen der Sicherungsvorrichtung 30 von der Fördereinrichtung 14 und der Betätigungshandhabe 16 erforderlich ist, dass beide gegeneinander versetzte Kopplungsabschnitte 40a, 42a gleichzeitig niedergedrückt werden müssen. Erst wenn dies geschehen ist, können die Teilschalen 33, 34 gegeneinander verschwenkt werden, um von der Fördereinrichtung 14 und der Betätigungshandhabe 16 getrennt zu werden und somit die Zugänglichkeit zur Betätigungshandhabe 16 wieder zu ermöglichen. Insbesondere für kleinere Kinder ist es zunächst schwer, zu durchschauen, dass die Sicherungsvorrichtung 30 aufgrund der Tatsache, dass sie die Fördereinrichtung 14 an einem unteren Rand 36 hintergreift, nicht einfach abgezogen werden kann, sondern aufgeschwenkt werden muss. Zudem ist es insbesondere für kleinere Kinder hinsichtlich des Bewegungsablaufes schwer, die beiden Entriegelungsflächen 40a, 42a gleichzeitig zu drücken und dann die erforderliche Aufschwenkbewegung durchzuführen.

Während die Ausgestaltung der Fig. 1a, 1b und 2 vorsieht, dass nicht nur die Fördereinrichtung 14 und die Betätigungshandhabe 16, sondern auch die Nasenolive 18 und damit auch die Austragöffnung 20 von der Sicherungsvorrichtung verdeckt sind, sind auch alternative Gestaltungen denkbar, bei denen die beiden Teilschalen 32, 34 in gekoppeltem Zustand eine gemeinsame Sicherungshülse bilden, die beidseitig offen ist und aus der sich die Nasenolive 18 nach außen erstreckt. Im Falle einer solchen Gestaltung ist es jedoch von Vorteil, wenn an der Innenseite der Teilschalen 32, 34 ein in Fig. 1b gestrichelt dargestellter Fortsatz 38 vorgesehen ist, der dann gemeinsam mit ebenfalls nach innen weisenden Fortsätzen 39, die auf einer Anschlagfläche der Fördereinrichtung 14 aufsitzen, die Verlagerbarkeit der Betätigungshandhabe 16 mechanisch verhindert.

Die Fig. 3a und 3b zeigen alternative Ausgestaltungen der Kopplungsvorrichtung.

Bei der Ausgestaltung der Fig. 3a sind ebenfalls zwei Kopplungsvorrichtungen 140, 142 vorgesehen, wobei diese jeweils einen an der Teilschale 32 angebrachten Schwenkschenkel 140a, 142a mit Ausnehmung 140b, 142b umfassen. An der anderen Teilschale 34 sind korrespondierende Nocken vorgesehen, die im dargestellten gekoppelten Zustand der Teilschalen 32, 34 in den Ausnehmungen 140b, 142b der Schwenkschenkel 140a, 142a angeordnet sind. Zum Entkoppeln müssen die Schwenkschenkel 140a, 142a in Richtung der Pfeile 4 aufeinander zu bewegt werden.

Bei der Gestaltung gemäß Fig. 3b ist an den Teilschalen 32, 34 eine Kopplungsvorrichtung 240 mit Kopplungsflanschen 241, 242 vorgesehen, wobei im Kopplungsflansch 241 eine Ausnehmung 241a vorgesehen ist und wobei am anderen Kopplungsflansch 242 ein Kopplungsfortsatz 242a mit einem aufgeweiteten Endbereich 242b vorgesehen ist. Im gekoppelten Zustand der Fig. 3b ragt der Kopplungsfortsatz 242a durch die Kopplungsdurchbrechung 241a hindurch. Da der aufgeweitete Endbereich 242b im untordierten Zustand des Fortsatzes 242a breiter ist als die Breite der Durchbrechung 241a, ist somit ein zuverlässig gekoppelter Zustand erzielbar. Zum Entkoppeln muss der Endbereich 242b Kopplungsfortsatz 242a um etwa 90° tordiert werden, bis der aufgeweitete Endbereich 242b durch die Durchbrechung 241a hindurchpasst. Eine solche Torsionsbewegung ist für Kinder sowohl hinsichtlich des Verständnisses als auch hinsichtlich der motorischen Fähigkeiten schwer zu realisieren.

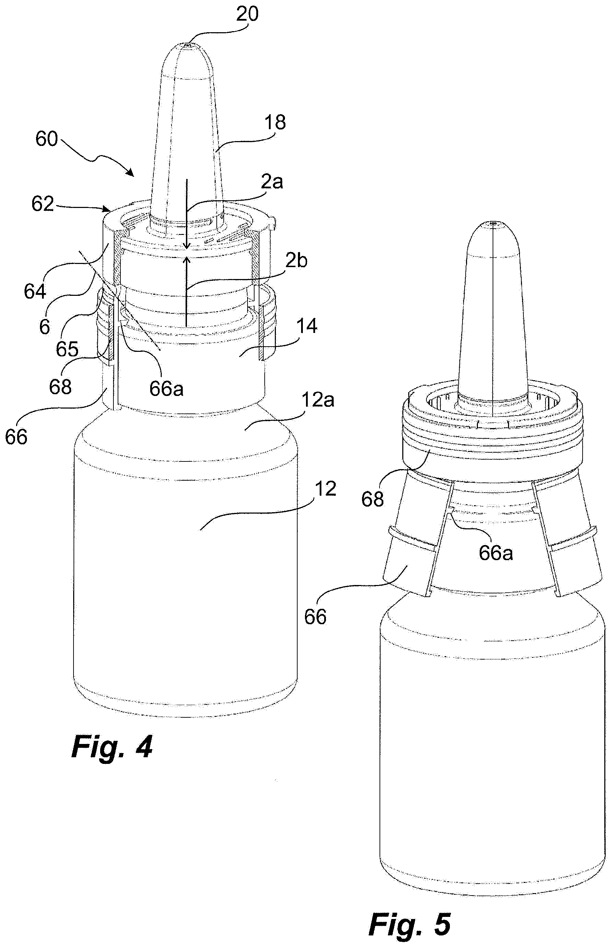

Die Fig. 4 bis 5 zeigen einen Spender gemäß einer nicht erfindungsgemäßen Variante. Der Grundaufbau dieses Spenders mit Mediumspeicher 12, Fördereinrichtung 14, Betätigungshandhabe 16 sowie Nasenolive 18 mit Austragöffnung 20 an deren distalen Ende ist gegenüber dem Spender der vorangegangenen Figuren vergleichbar. Die Sicherungsvorrichtung 60 gemäß dem Spender der Fig. 4 und 5 ist jedoch deutlich anders geartet. Diese Sicherungsvorrichtung 60 weist zwei separate Bauteile auf, nämlich ein Hauptsegment 62 sowie ein Schaltsegment 68. Das Hauptsegment 62 verfügt über einen Basisabschnitt 64, der in der Fig. 4 gut zu entnehmenden Weise fest mit der Betätigungshandhabe 16 verbunden ist. Der Basisabschnitt 64 weist eine ringartige Gestalt auf. Am unteren Ende des Basisabschnitts 64 finden sich insgesamt drei schwenkbare Sicherungsausleger 66, welche jeweils mit Filmscharnieren 65 am Basisabschnitt 64 um tangential ausgerichtete Schwenkachsen 6 verschwenkbar sind.

Während Fig. 4 eine Sicherungsschwenkposition der Sicherungsausleger 66 zeigt, zeigt Fig. 5 eine entsicherte Schwenkstellung. In Fig. 4 ist gut zu erkennen, dass an der Innenseite der Sicherungsausleger 66 Stützflächen 66a vorgesehen sind, die im gesicherten Zustand der Fig. 4 derart mit dem Grundkörper der Fördereinrichtung 14 zusammenwirken, dass eine Verlagerung der Betätigungshandhabe 16, die nur gemeinsam mit dem Basisabschnitt 64 verlagert werden kann, in Richtung des Pfeils 2a nicht möglich ist. Eine solche Bewegung ist erst dann möglich, wenn die Sicherungsausleger 66 ihre in Fig. 5 dargestellte Stellung einnehmen. Im gesicherten Zustand der Fig. 4 wird dieses Ausschwenken durch das Schaltsegment 68 verhindert. Das Schaltsegment 68 ist als Ring ausgestaltet, der außenseitig am Hauptsegment 62 vorgesehen ist und zwischen einer oberen Endlage der Fig. 5 und einer unteren Endlage der Fig. 4 verschiebbar ist. In der unteren Endlage der Fig. 4 sichert das Schaltsegment 68 die Sicherungsausleger 66 und verhindert deren Auslenkung nach außen. Es bedarf zunächst einer Verlagerung des Schaltsegments 68 in Richtung des Pfeils 2b nach oben, bis dieses die Position der Fig. 5 einnimmt. Erst dann können die Sicherungsausleger 66 nach außen verschwenkt werden und gestatten nachfolgend unbehindert eine Betätigung durch Verlagerung der Betätigungshandhabe und der gesamten Sicherungsvorrichtung 60 in Richtung des Pfeils 2a.

Der komplexe Bewegungsablauf ist zumindest für kleinere Kinder kaum zu bewerkstelligen, insbesondere da die Bewegungsrichtung des Schaltsegments 68 zum Entsichern der Sicherungsvorrichtung 60 in gegenüber der eigentlichen Betätigungsrichtung 2a entgegengesetzter Richtung 2b zu erfolgen hat.

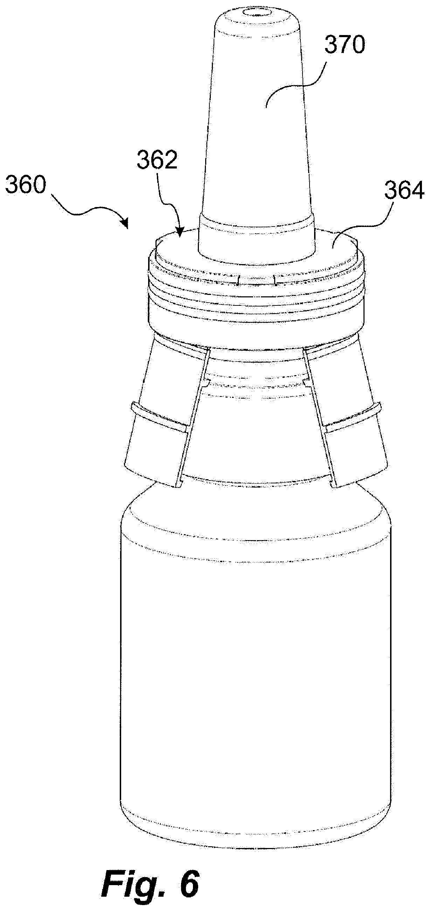

Fig. 6 zeigt eine Abwandlung der Ausgestaltung der Fig. 4 und 5, die sich dadurch unterscheidet, dass hier der Basisabschnitt 364 des Hauptsegments 362 der Sicherungsvorrichtung 360 nicht fest mit der Betätigungshandhabe 16 verbunden ist. Stattdessen umfasst die Sicherungsvorrichtung im Falle der Ausgestaltung der Fig. 6 auch einen Kappenabschnitt 370, der die Austragöffnung 20 überdeckt. Bei dieser Gestaltung ist vorgesehen, dass nach Entsicherung in Art, wie zu den Fig. 4 und 5 beschrieben, die Sicherungsvorrichtung 360 als Ganzes und einschließlich des Kappenabschnitts 370 abgenommen wird, um dann einen Austragvorgang zu gestatten.

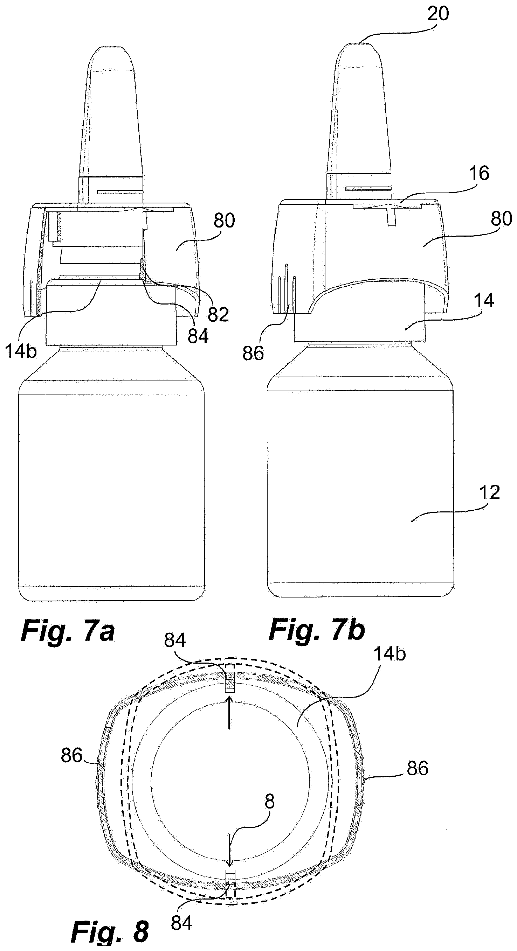

Die Fig. 7a, 7b und 8 zeigen eine Ausgestaltung einer nicht erfindungsgemäßen Variante. Figur 7a zeigt dabei zur Verdeutlichung eine teilgeschnittene Darstellung. Wiederum weist der Grundaufbau des Spenders die gleichen Merkmale wie bei den vorangegangenen Spendern auf, nämlich einen Medienspeicher 12, an dem sich eine Fördereinrichtung 14, eine hierzu gehörige Betätigungshandhabe 16 sowie eine Nasenolive 18 mit Austragöffnung 20 anschließen.

Die Besonderheit bei der Ausgestaltung gemäß der Fig. 7a, 7b und 8 liegt in einer Sperrvorrichtung 80, die an der Betätigungshandhabe 16 angebracht ist. Diese Sperrvorrichtung 80 verfügt in der Fig. 7a entnehmbaren Weise über einen ringförmigen Aufbau, an dessen Innenseite eine Klinke 82 mit einer Anschlagfläche 84 vorgesehen ist. Wie anhand der Schnittdarstellung der Fig. 8 erkennbar, überdeckt diese Klinke 82 mit der Anschlagfläche 84 im gesperrten Ausgangszustand der Sperrvorrichtung 80 eine Anschlagfläche 14b der Fördereinrichtung 14. Ein Niederdrücken der Betätigungshandhabe 16 ist daher nicht möglich.

Um einen Austragvorgang durch Niederdrücken der Betätigungshandhabe 16 in Richtung des Pfeils 2a zu bewerkstelligen, muss die Sperrvorrichtung 80 verformt werden. Hierfür sind an gegenüberliegenden Seiten der Außenfläche der Sperrvorrichtung 80 Entsperrflächen 86 vorgesehen. Durch eine Kraftbeaufschlagung dieser Entsperrflächen aufeinander zu wird eine Verformung der Sperrvorrichtung 80 bewirkt, wie sie in Fig. 8 gestrichelt dargestellt ist. Die Bewegung der Entsperrflächen 86 in Richtung 8a aufeinander zu führt gleichzeitig zu einer Bewegung der Sperrklinken 82 in Richtung der Pfeile 8 voneinander weg. Hierdurch werden die Sperrklinken 82 ausreichend weit nach außen verlagert, dass anschließend ein Niederdrücken der Betätigungshandhabe 16 möglich ist.

Obwohl das Konzept dieser Sperrvorrichtung sehr einfach ist, ist es für Kinder sehr schwer zu handhaben. Dies liegt insbesondere daran, dass zumindest kleinere Kinder bereits beide Hände benötigen, um hierdurch die Entsperrflächen 86 aufeinander zuzudrücken. Es gelingt ihnen daher jedoch nicht, nach einer derart bewirkten Entsperrung die Betätigungshandhabe 16 mitsamt der Sperrvorrichtung 80 niederzudrücken, so dass ein Austragvorgang nicht bewirkt wird.

## Patentansprüche

1. Spender zum Austrag eines flüssigen oder pulverförmigen Mediums mit
- einem Mediumspeicher (12),
- einer Austragöffnung (20) und
- einer Fördereinrichtung (14) zur Förderung von Medium vom Mediumspeicher (12) zur Austragöffnung (20),
wobei
- die Fördereinrichtung (14) über eine gegenüber dem Mediumspeicher (12) in einer Betätigungsrichtung (2a) beweglichen Betätigungshandhabe (16) verfügt, mittels derer die Fördereinrichtung (14) zum Zwecke des Austrags betätigt werden kann, wobei eine Sicherungsvorrichtung (30) vorgesehen ist, die
- aus mindestens zwei Teilschalen (32, 34) besteht, die in einem Sicherungszustand durch mindestens eine zum wiederholten Entkoppeln und Koppeln ausgebildete Kopplungsvorrichtung (40, 42; 140, 142; 241) derart miteinander verbunden, dass sie gegeneinander unbeweglich sind,
wobei
- die Sicherungsvorrichtung (30) an der Fördereinrichtung (14) des Spenders in einer Sicherungsposition derart anbringbar ist, dass im Sicherungszustand der Sicherungsvorrichtung (30) die Zugänglichkeit und/oder Beweglichkeit der Betätigungshandhabe (16) verhindert wird, und
- durch Lösen der Kopplungsvorrichtung (40, 42; 140, 142; 241) ein Freigabezustand der Sicherungsvorrichtung (30) erzielbar ist, in dem die Teilschalen (32, 34) gegeneinander beweglich sind, so dass die Sicherungsvorrichtung (30) von der Fördereinrichtung trennbar ist und so die Zugänglichkeit oder Beweglichkeit der Betätigungshandhabe (16) herstellbar ist, und
- die Kopplungsvorrichtung (40, 42; 140, 142; 241) durch formschlüssig wirkende Kopplungsabschnitte (40a, 42a; 142a, 142b; 241a, 242a, 242b) an den beiden durch die Kopplungsvorrichtung gekoppelten Teilschalen gebildet ist, die durch abschnittsweises Verformen der Kopplungsabschnitte (40a, 42a; 142a, 142b; 241a, 242a, 242b) einer oder beider Teilschalen aus einem gekoppelten in einen entkoppelten Zustand überführt werden können, **dadurch gekennzeichnet, dass** die Sicherungsvorrichtung (30) im angebrachten Zustand eine zumindest einseitig offene Hülse ist, die den Spender partiell im Bereich der Fördereinrichtung (14) umgibt.

2. Spender nach Anspruch 1,
**dadurch gekennzeichnet, dass**
die zwei Teilschalen (32, 34) durch ein Scharnier (33) miteinander schwenkbar verbunden sind.

3. Spender nach Anspruch 1 oder 2,
**dadurch gekennzeichnet, dass**
mindestens zwei getrennt handhabbare Kopplungsvorrichtungen (40, 42; 140, 142) vorgesehen sind, die durch Verformung der Kopplungsabschnitte (40a, 42a; 142a, 142b) beider Teilschalen in gleicher Richtung oder die durch Verformung von zwei Kopplungsabschnitten der gleichen Teilschale in gegenläufige Richtung entkoppelbar sind.

4. Spender nach einem der vorstehenden Ansprüche,
**dadurch gekennzeichnet dass**
die Kopplungsvorrichtung (240) einen Kopplungsdurchbrechung (241a) an einer Teilschale (32) und einen die Kopplungsdurchbrechung im gekoppelten Zustand durchdringenden Kopplungsfortsatz (242a, 242b) an der anderen Teilschale (34) aufweist, wobei der Kopplungsfortsatz (242a, 242b) derart geformt ist, dass er zum Hindurchführen durch die Kopplungsdurchbrechung (241a) zum Zwecke des Entkoppelns tordiert werden muss.

## Claims

1. Dispenser for dispensing a liquid or pulverulent medium, having
- a medium store (12),
- a dispensing opening (20) and
- a delivery mechanism (14) for delivering medium from the medium store (12) to the dispensing opening (20),
wherein
- the delivery mechanism (14) has an actuating handle (16), which can be moved in an actuating direction (2a) in relation to the medium store (12) and by means of which the delivery mechanism (14) can be actuated for dispensing purposes,
wherein
a protective device (30) is provided, and this safeguarding device
- comprises at least two sub-shells (32, 34) which, in a safeguarding state, are connected to one another, by at least one coupling device (40, 42; 140, 142; 241) designed for repeated uncoupling and coupling operation, such that they cannot be moved in relation to one another,
wherein
- the protective device (30) can be fitted on the delivery mechanism (14) of the dispenser in a safeguarding position such that, in the safeguarding state of the protective device (30), accessibility to and/or movability of the actuating handle (16) are/is prevented, and
- release the coupling device (40, 42; 140, 142; 241) an enable state of the protective device is achievable, in which the sub-shells (32, 34) can be moved in relation to one another, and therefore the protective device (30) can be separated from the delivery mechanism and it is thus possible to establish accessibility to or movability of the actuating handle (16), and
- the coupling device (40, 42; 140, 142; 241) is formed by form-fitting coupling portions (40a, 42a; 142a, 142b; 241a, 242a, 242b) on the two sub-shells, which are coupled by the coupling device, and these can be transferred from a coupled state into an uncoupled state by partial deformation of the coupling portions (40a, 42a; 142a, 142b; 241a, 242a, 242b) of one or both sub-shells,
**characterized in that** the protective device (30), in the fitted state, forms a sleeve which is open at least on one side and encloses the dispenser partially in the region of the delivery mechanism (14).

2. Dispenser according to Claim 1,
**characterized in that**
the two sub-shells (32, 34) are connected to one another in a pivotable manner by a hinge (33).

3. Dispenser according to Claim 1 or 2,
**characterized by**
the provision of at least two separately handleable coupling devices (40, 42; 140, 142), which can be uncoupled by virtue of the coupling portions (40a, 42a; 142a, 142b) of the two sub-shells being deformed in the same direction or which can be uncoupled by virtue of two coupling portions of the same sub-shell being deformed in opposite directions.

4. Dispenser according to one of the preceding claims,
**characterized in that**
the coupling device (240) has a coupling aperture (241a) on one sub-shell (32) and a coupling extension (242a, 242b) on the other sub-shell (34), said coupling extension passing through the coupling aperture in the coupled state, wherein the coupling extension (242a, 242b) is formed such that it has to be twisted in order to be guided through the coupling aperture (241a) for uncoupling purposes.

## Revendications

1. Distributeur pour délivrer un milieu liquide ou pulvérulent, comprenant :
- un dispositif de stockage de milieu (12),
- une ouverture de décharge (20) et
- un dispositif de transport (14) pour transporter le milieu depuis le dispositif de stockage de milieu (12) jusqu'à l'ouverture de décharge (20),
- le dispositif de transport (14) disposant d'une manette d'actionnement (16) déplaçable dans une direction d'actionnement (2a) par rapport au dispositif de stockage de milieu (12), au moyen de laquelle le dispositif de transport (14) peut être actionné en vue de la décharge,
un dispositif de fixation (30) étant prévu, lequel se compose d'au moins deux coques partielles (32, 34) qui sont connectées l'une à l'autre dans un état de fixation par au moins un dispositif d'accouplement (40, 42 ; 140, 142 ; 241) réalisé de manière à permettre un désaccouplement et un accouplement répétés, de telle sorte qu'elles ne puissent pas être déplacées l'une par rapport à l'autre,
- le dispositif de fixation (30) pouvant être amené dans une position de fixation sur le dispositif de transport (14) du distributeur de telle sorte que dans l'état de fixation du dispositif de fixation (30), il ne soit pas possible d'accéder à et/ou de déplacer la manette d'actionnement (16), et
- un état de libération du dispositif de fixation (30) pouvant être obtenu par desserrage du dispositif d'accouplement (40, 42 ; 140, 142 ; 241), dans lequel état de libération les coques partielles (32, 34) peuvent être déplacées l'une par rapport à l'autre de telle sorte que le dispositif de fixation (30) puisse être séparé du dispositif de transport et que l'on puisse ainsi accéder à ou déplacer la manette d'actionnement (16), et
- le dispositif d'accouplement (40, 42 ; 140, 142 ; 241) étant formé par des portions d'accouplement (40a, 42a ; 142a, 142b ; 241a, 242a, 242b) agissant par engagement par correspondance de formes sur les deux coques partielles accouplées par le dispositif d'accouplement, qui peuvent être amenées par déformation partielle des portions d'accouplement (40a, 42a ; 142a, 142b ; 241a, 242a, 242b) d'une ou des deux coques d'un état accouplé dans un état désaccouplé,
**caractérisé en ce que**
le dispositif de fixation (30), dans l'état monté, est au moins une douille ouverte d'un côté qui entoure le distributeur en partie dans la région du dispositif de transport (14).

2. Distributeur selon la revendication 1, **caractérisé en ce que**
les deux coques (32, 34) sont connectées l'une à l'autre de manière pivotante par le biais d'une charnière (33).

3. Distributeur selon la revendication 1 ou 2,
**caractérisé en ce**
**qu'**au moins deux dispositifs d'accouplement manipulables séparément (40, 42 ; 140, 142) sont prévus, lesquels peuvent être désaccouplés par déformation des portions d'accouplement (40a, 42a ; 142a, 142b) des deux coques partielles dans le même sens ou par déformation de deux portions d'accouplement de la même coque partielle dans le sens inverse.

4. Distributeur selon l'une quelconque des revendications précédentes,
**caractérisé en ce que**
le dispositif d'accouplement (240) présente une interruption d'accouplement (241a) au niveau d'une coque partielle (32) et une saillie d'accouplement (242a, 242b) traversant l'interruption d'accouplement dans l'état accouplé au niveau de l'autre coque partielle (34), la saillie d'accouplement (242a, 242b) étant formée de telle sorte qu'elle doive être tordue pour permettre le guidage à travers l'interruption d'accouplement (241a) dans le but du désaccouplement.
